Europäisches Patentamt

European Patent Office

Office européen des brevets

(11) Publication number: **0 407 574 A1**

## EUROPEAN PATENT APPLICATION
### published in accordance with Art. 158(3) EPC

(21) Application number: 88909830.7

(22) Date of filing: **10.11.88**

(86) International application number: **PCT/JP88/01129**

(87) International publication number: **WO 90/05193 (17.05.90 90/11)**

(51) Int. Cl.5: **C12Q 1/40**

(43) Date of publication of application: **16.01.91 Bulletin 91/03**

(84) Designated Contracting States: **DE FR GB**

(71) Applicant: **ORIENTAL YEAST CO., LTD.**
**6-10, Azusawa 3-chome Itabashi-ku Tokyo 174(JP)**

(72) Inventor: **TAKATA, Shigeru**
**4-27, Minamiasahigaoka-cho**
**Tondabayashi-shi**
**Osaka-fu 584(JP)**
Inventor: **FUJITA, Tuyosi**
**103, 2-16, Satsukigaoka 2-chome**
**Ikeda-shi Osaka-fu 563(JP)**
Inventor: **TAKAGAHARA, Isamu**
**3-59, Fushimidai 5-chome Inagawa-cho**
**Kawabe-gun Hyogo-ken 666-02(JP)**

(74) Representative: **Thomsen, Dieter, Dr.rer.nat.**
**Kaiser-Ludwig-Platz 6**
**D-8000 München 2(DE)**

(54) METHOD FOR IMPROVING SENSITIVITY AND ACCURACY OF ANALYSIS.

(57) A method for improving sensitivity and/or accuracy of analysis by using a readily soluble clathrate compound such as glucosyl-α-cyclodextrin, maltosyl-α-cyclodextrin or the like in an analytic system is disclosed, α-cyclodextrin has been used in an analytic system for improving analytical sensitivity, but it has the defect that, since the maximum sensitivity pH must be shifted to a desired pH in conducting analysis, a large amount of α-cyclodextrin is necessary and that a reagent containing a large amount of α-cyclodextrin is liable to cause crystallization and solidification during storage, thus becoming non-usable. The readily soluble clathrate compounds such as glucosyl-α-cyclodextrin or maltosyl-α-cyclodextrin permit the sensitivity and accuracy to be improved by using them only in a small amount and prevent crystallization and solidification of a reagent containing them during storage.

## FIG. 1

RELATIONSHIP BETWEEN THE CONCENTRATION
OF THE READILY-SOLUBLE INCLUSION
COMPOUND ADDED AND THE ABSORBANCE

CONCENTRATION OF THE READILY-SOLUBLE
CLATHRATE COMPOUND (mmol/l)

**EP 0 407 574 A1**

Oriental Yeast Co., Ltd.

Tokyo / Japan

13. Aug. 1989

TITLE MODIFIED
see front page

## Method for Improving Analytical Sensitivity and Analytical Accuracy

The present invention relates to a method for improving analytical sensitivity and/or accuracy, and is significantly used in such technical fields as chemical analysis, enzymatic analysis, immunological analysis, etc.

Therefore, the present invention may be applied to reagents for analysis and measurement and clinical diagonostic agents.

### Prior Art

Heretofore, where enzyme activity is to be measured, methods using nitrophenol derivatives as indicators are known, but with the methods using nitrophenol type indicators, since the indicators do not completely dissociate at a pH where enzyme exhibits the maximum activity, a drop in sensitivity could not be avoided.

Further, these indicators also had a disadvantage that the stability of absorbance against the vibration of pH in the vicinity of the pH to measure its enzyme activity.

In order to solve these disadvantages, a method for increasing analytical sensitivity in enzyme activity measurement by using cyclodextrin which is an inclusion compound, has been developed (Japanese Patent Application Laid-open Official Gazette No. Sho 58-501357), and more specifically, it has been disclosed that by using $\alpha$-cyclodextrin, the maximum sensitivity pH was changed from 7.3 to 7.0, thereby increasing the maximum sensitivity by about 50%, and that as a result it has become possible to measure $\alpha$-amylase activity in human serum.

Problems to be Solved by the Invention

However, with this method using cyclodextrin, in order to conduct measurement and analysis by shifting the maximum sensitivity pH to the desired pH, it is necessary to use a very large amount of cyclodextrin, and it is impossible to avoid a disadvantage that such a large amount of cyclodextrin exhibits an interefering effect on measurement of minor components such as components in the body fluids and thus accurate measurement becomes impossible.

Further, the cyclodextrin-containing reagent used therefor is extremely poor in stability to cause crystallization and solidification phenomena even when stored at room temperature and it cannot withstand use. Moreover, such a biological reagent must be refrigerated because the components would deteriorate if left at room temperature, but if refrigerated, the crystallization and solidification phenomena are promoted and there is a great problem in practice.

## Means for Solving the Problems

The present invention has been achieved in order to solve this disadvantage, and as the result of wide studies from various aspects, it has been concluded that the advantages of cyclodextrin cannot be neglected for analysis of vital components.

And, as the result of the intensive study for the purpose of improving the cyclodextrin method, it has been found out that its solubility is an important factor, and finally cyclodextrin having high solubility has become to draw attention.

Then, when $\alpha$-amylase was measured by using glucosyl-$\alpha$-cyclodextrin as a readily-soluble cyclodextrin and p-nitrophenol as an indicator, measurement results excellent in sensitivity and accuracy were obtained and furthermore it was confirmed that there is no problem with the storability of the reagent. Then, it was also confirmed that these excellent results may be exerted with all the inclusion compounds, and based on this new discovery, the present invention has thus been accomplished.

That is, the present invention is a method for improving sensitivity and accuracy of analysis, the important point thereof residing in the use of a readily-soluble inclusion compound having high solubility.

## Brief Description of the Drawing

Fig. 1 is a graph showing the relationship of the amount of the cyclodextrin derivative added and the increase of its absorbance in Example 1; and

Fig. 2 is a graph showing the change in absorbance against the pH change upon addition of the cyclodextrin derivative in Example 2.

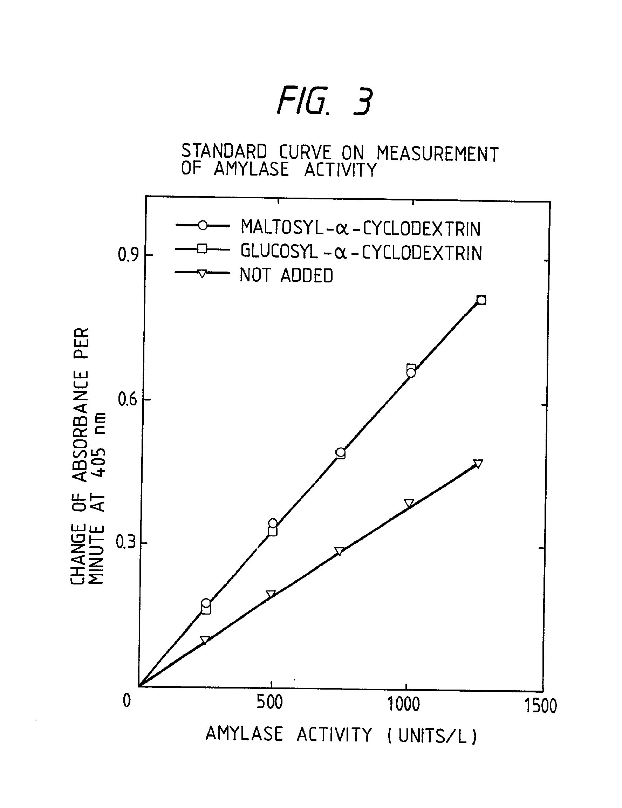

Further, Fig. 3 is a standard curve in the measurement of amylase activity in Example 3.

As the readily-soluble inclusion compound in the present invention, any readily-soluble inclusion compound excellent in solubility may be used, and as examples therefor, there may be exemplified sugar derivatives of cyclodextrin such as glucosyl-α-cyclodextrin, maltosyl-α-cyclodextrin etc.

The present invention may be widely used especially in chemical analytical methods, enzymatic analytical methods, immunological analytical methods which use a nitrophenol derivative as an indicator, and may be advantageously utilized in, for example, in addition to analysis of hydroxycinnamic acid using nitrophenol, analysis of amylase and glucosidase with nitrophenyl(oligo)glycoside as a substrate, analysis of galactosidase with nitrophenylgalactoside as a substrate, and also analysis of mannosidase, fucosidase and phosphatase using mannoside, fucoside and phosphate as substrates.

The principle of the enzyme analysis by the present invention is such that a p-nitrophenol sugar substrate is contacted with, for example, α-amylase, then, decomposed into p-nitrophenol and glucose by using α-glucosidase, glucoamylase etc., and the yellow color of the thus formed p-nitrophenol is read by absorbance at 405 nm, thereby the activity of $\alpha$-amylase being measured. In the present invention, a readily-soluble inclusion compound is added in this measurement system, but the time of its addition is not particularly restricted, and the predetermined effect may be obtained at any time of the measurement system, and since there is utterly no need for delicate operations concerning the time of addition, it is particularly advantageous in the actual clinical scene with a great rush of work.

The readily-soluble inclusion compound is not needed to be used in a great amount as in the prior art method, and for example, in the case of maltosyl- or glucosyl-$\alpha$-cyclodextrin, the intended purpose may be sufficiently attained with the usage of 0.05 - 100 mmol/l , preferably about 1 - 20 mmol/l . Therefore, the vital components present in extremely slight amounts may be quantitatively analyzed accurately and rapidly.

On conducting analysis in practice by using the method of the present invention, it is convenient to previously prepare an analytical reagent for amylase by dissolving a p-nitrophenol-combined amylase substrate, a readily-soluble inclusion compound, $\alpha$-glucosidase, glucoamylase etc. in a buffer. This reagent is completely different from the prior art reagents, and does not crystallize out or solidify even if refrigerated and stored for a prolonged time, and thus is extremely excellent.

For example, for analyzing amylase in the human serum or urine by using this reagent, the following operations may sufficiently be conducted. First, the reagent is placed in a cuvette and heated to 37°C; a sample is added thereto and mixed; the color development of p-nitrophenol is read by absorbance at about 405 nm, and the amylase activity is measured in a conventional manner.

The present invention is characterized by the point that the sensitivity and accuracy are improved by using an inclusion compound readily soluble in water. The details of its mechanism remain to be solved by future researches, but for the present, it is presumed that by forming an inclusion complex with the inclusion compound and the nitrophenol derivative contained in the diagnostic reagent, its absorbance is increased and further the absorbance against the pH change is stabilized.

The examples of the present invention are described in detail below.

Example 1   Increase in Absorbance with Cyclodextrin
            Derivative

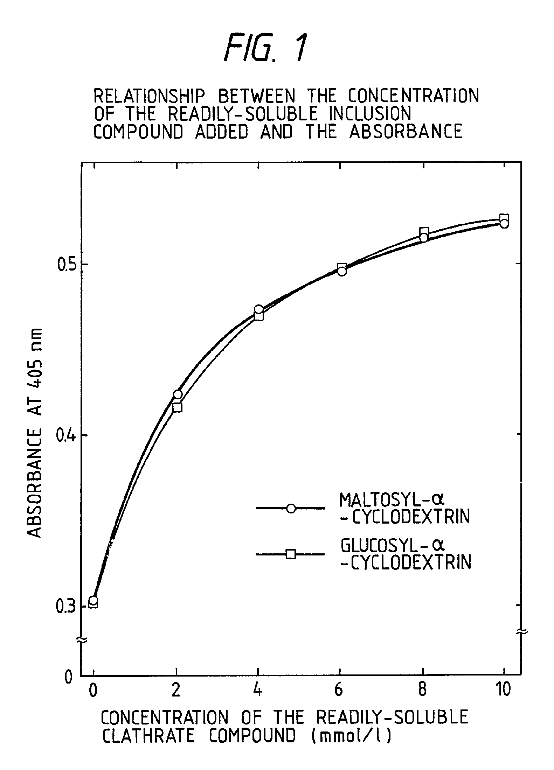

To 100 mmol/l PIPES buffer (pH 7.0) and 34 µmol/l para-nitrophenol was added glucosyl-$\alpha$-cyclodextrin or maltosyl-$\alpha$-cyclodextrin at a proportion of 0 - 10 mmol/l respectively, and the absorbance at 405 nm was measured, to obtain the results of Fig. 1.

## Example 2   Stabilization of Absorbance with Cyclodextrin
###      Derivative

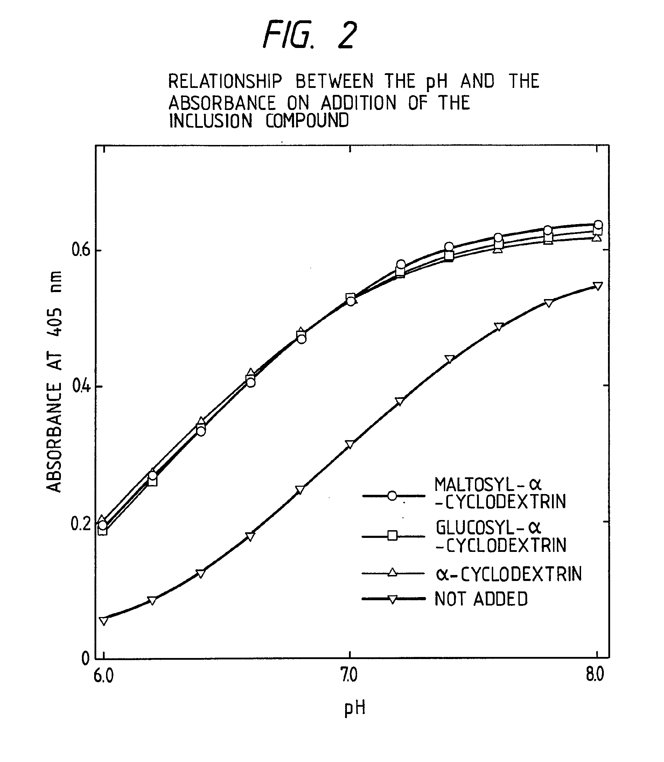

To 100 mmol/l PIPES buffer and 34 μmol/l para-nitrophenol was added glucosyl-α-cyclodextrin or maltosyl-α-cyclodextrin in 10 mmol/l respectively, and relationship between pH and absorbance changes was measured, to obtain the results of Fig. 2.

## Example 3   Measurement of Amylase Activity

To 100 mmol/l PIPES buffer (pH 7.0) containing 1 mmol/l benzilydene-paranitrophenylmaltoheptaoside, 20 U/ml glucoamylase, 10 U/ml α-glucosidase, 20 mmol/l NaCl and 2 mmol/l calcium acetate was added glucosyl-α-cyclodextrin or maltosyl-α-cyclodextrin in 10 mmol/l, and the amylase activity was measured at 37°C, to obtain the results of Fig. 3.

As evident also from these results, it can be seen that by using the readily-soluble inclusion compound, the amylase activity may be measured accurately and at high sensitivity.

## Effects of the Invention

As has been described above, the method for improving analytical sensitivity and accuracy of the present invention exerts a remarkable effect that the analytical sensitivity of the indicator nitrophenol derivative and the stability against the pH change are extremely increased by using a cyclodextrin derivative readily soluble in water.

That is, the high accuracy detection of the intended substance in a very slight amount in the specimen becomes possible.  Therefore, the present invention is effective

8

especially in analysis of various minor components, and especially effective in the field of clinical diagnosis.

EP 0 407 574 A1

13. Aug. 1989

## CLAIMS

1. A method for improving sensitivity and/or accuracy of analysis which is characterized by using a readily-soluble inclusion compound in an analytical system.

2. The method as described in Claim 1 which is characterized by that the analytical system is a clinical diagnostic agent which contains an indicator having a convertible chemical or physical state and components necessary for conducting the desired analysis.

3. The method as described in Claim 1 wherein the readily-soluble inclusion compound is glucosyl-$\alpha$-cyclodextrin and/or maltosyl-$\alpha$-cyclodextrin.

4. The method as described in Claim 2 wherein the concentration of glucosyl-$\alpha$-cyclodextrin and/or maltosyl-$\alpha$-cyclodextrin is 1 - 20 mmol/l .

5. The method as described in Claim 1 wherein the indicator is para-nitrophenol.

6. The method as described in Claim 1 wherein the pH of the diagnostic reagent is 6 - 8.

7. The method as described in Claim 1 wherein the object to be analyzed is amylase.

# *FIG. 1*

RELATIONSHIP BETWEEN THE CONCENTRATION
OF THE READILY-SOLUBLE INCLUSION
COMPOUND ADDED AND THE ABSORBANCE

# FIG. 2

## RELATIONSHIP BETWEEN THE pH AND THE ABSORBANCE ON ADDITION OF THE INCLUSION COMPOUND

FIG. 3

STANDARD CURVE ON MEASUREMENT
OF AMYLASE ACTIVITY

# INTERNATIONAL SEARCH REPORT

International Application No   PCT/JP88/01129

**I. CLASSIFICATION OF SUBJECT MATTER** (if several classification symbols apply, indicate all) °

According to International Patent Classification (IPC) or to both National Classification and IPC

Int.Cl⁴    C12Q1/40

**II. FIELDS SEARCHED**

| Minimum Documentation Searched [7] | |
|---|---|
| Classification System ı | Classification Symbols |
| IPC | C12Q1/26-1/52 |

Documentation Searched other than Minimum Documentation
to the Extent that such Documents are Included in the Fields Searched ⁸

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | JP, A, 58-501357 (American Hospital Supply Corporation) 18 August 1983 (18. 08. 83) & US, A, 4451563 & EP, A, 86821 | 1-2, 5-7 |
| Y | JP, B2, 62-138199 (Hitachi Chemical Co., Ltd.) 20 June 1987 (20. 06. 87) (Family: none) | 1-2, 5-7 |
| Y | JP, B2, 57-102198 (Toyobo Co., Ltd.) 25 June 1982 (25. 06. 82) (Family: none) | 1-2, 6-7 |

\* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| January 25, 1989 (25. 01. 89) | January 30, 1989 (30. 01. 89) |
| International Searching Authority | Signature of Authorized Officer |
| Japanese Patent Office | |

Form PCT/ISA/210 (second sheet) (January 1985)